# EUROPEAN PATENT SPECIFICATION

(11) **EP 3 125 841 B1**
(45) Date of publication and mention of the grant of the patent: **13.05.2020**
(21) Application number: 15720458.7
(22) Date of filing: 01.04.2015
(51) Int. Cl.: A61F 9/06, B23K 9/095, B23K 9/32, F16P 1/06, B23K 9/10, G01R 15/20

(54) **WELDING AND/OR CUTTING UNIT WITH SENSOR FOR DETECTING/MEASURING THE WELDING AND/OR CUTTING CURRENT**
SCHWEISS- UND/ODER SCHNEIDEINHEIT MIT EINEM SENSOR ZUR ERFASSUNG/MESSUNG VOM SCHWEISS- UND/ODER SCHNEIDSTROM
UNITÉ DE SOUDAGE ET/OU DE DÉCOUPAGE AVEC UN CAPTEUR POUR DÉTECTER/MESURER LE COURANT DE SOUDAGE ET/OU DE DÉCOUPAGE

(30) Priority: 04.04.2014 IT VI20140090
(43) Date of publication of application: 08.02.2017
(73) Proprietor: Trafimet Group S.p.A., 36020 Castegnero (VI) (IT)
(72) Inventor: SIMIONI, Ugo, I-36100 Vicenza (VI) (IT); IMI, Attilio, I-33010 Pagnacco (UD) (IT)
(74) Representative: Ziliotto, Tiziano
(86) International application number: PCT/IB2015/052409
(87) International publication number: WO 2015/151051

(56) References cited:
- EP-A1- 2 682 762
- DE-A1-102012 104 348
- US-A1- 2006 232 902
- US-A1- 2010 301 018

## Description

### TECHNICAL FIELD OF THE INVENTION

The present invention concerns the field of welding systems. More particularly, the present invention concerns the field of sensors for welding and/or cutting systems. In greater detail, the present invention describes a welding and/or cutting unit according to the preamble of claim 1 (see for example US 2010/301018 A1).

### PRIOR ART

Several welding and/or cutting current sensors for welding and/or cutting systems are known in the art. In many cases, however, the known sensors pose some drawbacks. They are often cumbersome and therefore difficult to install and expensive.

Therefore, it is the object of the present invention to provide a welding and/or cutting unit comprising a welding and/or cutting current sensor that at least partially solves the problems mentioned above. In particular, it is the object of the present invention to provide a welding system in which the welding and/or cutting current sensor is small-sized, easy to install and economical to produce.

### SUMMARY OF THE INVENTION

The present invention is based on the idea of providing a current sensor for welding and/or cutting systems that comprises a small-sized Hall probe.

According to the present invention, a welding and/or cutting unit is defined in claim 1.

The sensor is precise, occupies a small space and makes it possible to detect the passage of welding current and to measure its intensity.

According to a further embodiment of the present invention, a welding and/or cutting system comprising such welding and/or cutting unit is defined in claim 8.

According to a further embodiment of the present invention, the ferromagnetic circuit comprises one or more C-shaped elements.

According to a further embodiment of the present invention, said one or more C-shaped elements at least partially envelop said one or more power supply cables of the torch or parts of the torch body.

According to a further embodiment of the present invention, the current sensor that is such as to comprise a container where the ferromagnetic circuit can be closed.

According to a further embodiment of the present invention, the current sensor is suited to be connected to a microprocessor included in the welding system, in such a way as to signal to the first microprocessor the presence of current flowing through said one or more cables of said connection element.

According to a further embodiment of the present invention, the microprocessor is suited to automatically recognize the operating mode of the torch, for example 2T or 4T mode, based on the signal received from the current sensor and on the position of the push button.

According to a further embodiment of the present invention, the welding and/or cutting system is provided, in which the darkening of a protection screen, for example an LCD filter, included in the protection unit, is a function of the current measured by the current sensor.

### BRIEF DESCRIPTION OF THE DRAWINGS

The present invention will be described with reference to the attached drawings, in which the same reference numbers and/or signs indicate the same and/or similar and/or corresponding parts of the system.
Figure 1 schematically shows a welding and/or cutting system according to an embodiment of the present invention.
Figures 2A and 2B schematically show a detail of the welding system shown in Figure 1.
Figure 3 schematically shows a further detail of the welding system shown in Figure 1, in 3D (Figure 3A) and in a sectional view (Figure 3B).
Figure 4 schematically shows a further detail of the welding system shown in Figure 1.
Figure 5 schematically shows a further detail of the welding system shown in Figure 1.

### DETAILED DESCRIPTION OF THE INVENTION

The present invention is described here below with reference to specific embodiments illustrated in the attached drawings. However, the present invention is not limited to the specific embodiments disclosed in the following detailed description and illustrated in the figures, but, rather, the embodiments described below simply exemplify several aspects of the present invention, the scope of which are defined in the claims.

Further modifications and variants of the present invention will be clear to the expert in the art.

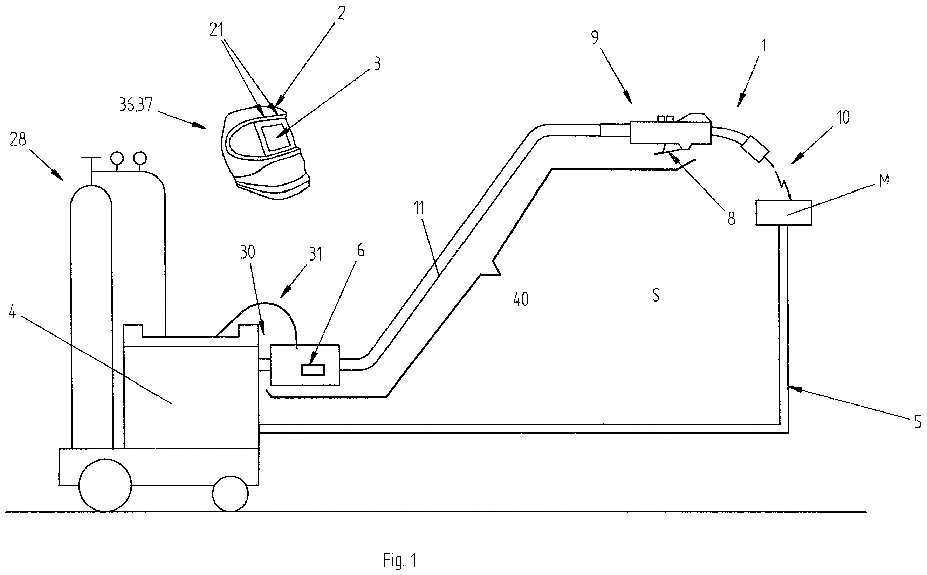

Figure 1 schematically shows a welding and/or cutting system according to an embodiment of the present invention.

The welding and/or cutting system according to the present invention may comprise welding machines of the MMA, TIG, MIG/MAG type, plasma welding machines or with electrodes with or without protective gas. In order to simplify the explanation, the description provided here below makes reference to a welding system, but it applies as well to a cutting system.

The welding system shown in the figure may comprise three fundamental units: a main unit, a protection unit and a data collection/transmission unit.

The main unit comprises a generator 4 to which a gas cylinder 28 and/or a cooling circuit is/are connected. The main unit furthermore comprises a welding unit that in turn comprises a welding torch 1, a ground cable 5 that connects the generator 4 to the element to be welded M and a connection element 40.

The connection element 40 comprises a connector 30, a cable bundle 11, pipes for the passage of water in case a cooling circuit for the torch 1 is provided, and pipes for the passage of pressurized air in the case of a torch for plasma cutting or for the passage of a gas or a gas mixture in the case where the welding system is designed to include the use of the latter.

The cable bundle 11 connects the torch 1 to the connector 30 and the connector 30 connects the cable bundle 11, and therefore the torch 1, to the generator 4. The cable bundle 11 furthermore comprises at least one or more control cables 11a of the torch and one or more power supply cables 11b of the torch 1, not shown in the figure.

The welding torch 1 comprises a push button 8 for starting/stopping the welding process and a grip 9 intended to allow the operator to handle the torch 1 conveniently.

The ground cable 5 closes the circuit made up of generator, torch, element to be welded M and thus allows the welding operations to be started.

The welding unit furthermore comprises a control system 6 with first transmission/reception means 7. According to the present invention, the control system 6 is mounted between the connector 30 and the cable bundle 11. The first transmission/reception means 7 preferably comprise a radiofrequency unit. Said radiofrequency unit is preferably of the Bluetooth® type. The first transmission/reception means 7 allow data and/or information to be transferred from the main unit to the other components of the welding system and/or to any external device operating with Bluetooth® technology, for example a tablet or similar devices.

The power supply to the control system 6 is ensured by preferably rechargeable electrical accumulators. Said recharge can be obtained by using a battery charger that can be connected to the control system 6 or using the power supply cable 11b that feeds the torch 1 and/or external generators. The protection unit comprises a protective helmet 2 that the operator can wear during the welding operations. The protection unit furthermore comprises second transmission/reception means 12, not shown in the figure, and a protection screen 3, preferably an LCD filter.

The second transmission/reception means 12 preferably comprise a radiofrequency unit. Preferably, said radiofrequency unit is of the Bluetooth® type. The second transmission/reception means 12 allow data and/or information to be transferred from the protection unit to the other components of the welding system and/or to any external device operating with Bluetooth® technology, for example an iPad or similar devices.

The power supply to the devices included in the protection unit is obtained by means of rechargeable and/or replaceable electrical accumulators. The data collection/transmission unit C comprises the sensors 41, 42, 43, 15 and a data collection/transmission system 27. The data collection/transmission system 27 can advantageously be integrated in the control system 6 or can be mounted in the welding system, in the most convenient position.

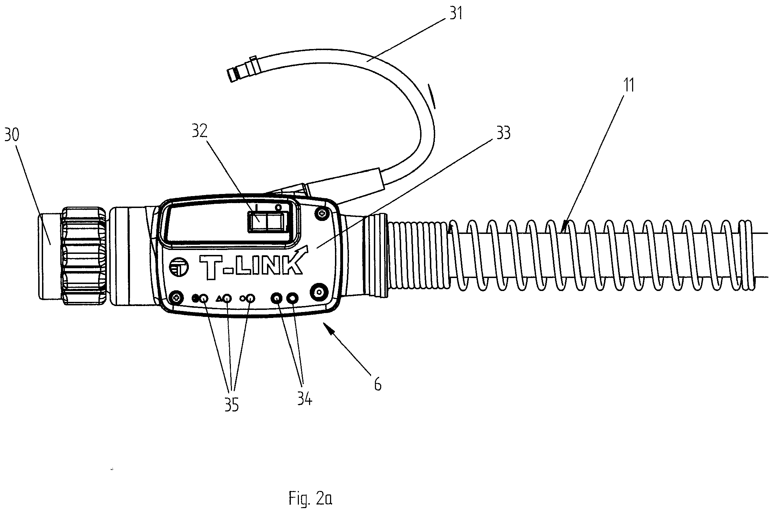

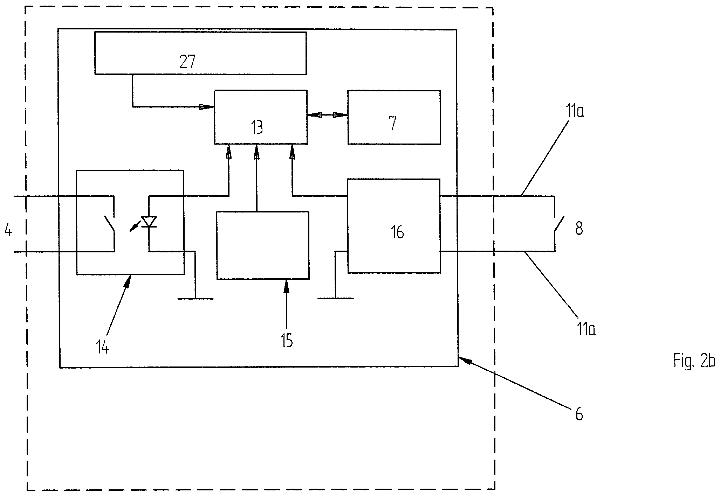

Figures 2A and 2B schematically show a detail of the welding system shown in Figure 1.

More particularly, Figure 2A schematically shows a front view of the control system 6. The control system 6 is mounted between the connector 30 and the cable bundle 11. The control system 6 comprises a control panel 33 that in turn comprises an on/off button 32 suited to switch the control system 6 on/off, warning lights 34 that signal the status of the control system 6, push buttons 35 by means of which the operator can set some parameters related to the welding process.

The pipe 31 connects the control system to the cooling circuit of the torch 1, where provided.

Figure 2B shows a block diagram of the components of the control system 6.

When the operator holds the torch 1 and presses the button 8 to start the welding process, an electrical signal is transferred from the button 8 to the control system 6, by means of one or more control cables 11a. The microprocessor 13, included inside the control system 6, comprises an interface for connection with the button 8. The connection interface preferably comprises a low-pass filter 16. The low-pass filter 16 is preferably of the capacitive type. Preferably, there is one low-pass filter 16 for each one of the one or more control cables 11a of the cable bundle 11. The low-pass filter 16 makes it possible to filter the high frequency disturbances that otherwise would alter the signal received by the microprocessor 13. The disturbances get into the one or more control cables 11a of the cable bundle 11 by induction, due to the proximity of the one or more control cables 11a to the power supply cables 11b of the torch 1 that are also present in the cable bundle 11.

When the microprocessor 13 receives the signal intended to communicate that the button 8 has been pressed, it transmits the information to the protection unit, as better explained below, and then sends the signal to the generator 4. At this point the generator 4 can start to supply welding current through the cable bundle 11. The connection interface between the microprocessor 13 and the generator 4 preferably comprises a galvanic isolation element 14 for each one of the one or more control cables 11a. The galvanic isolation element 14 may comprise a relay, preferably in the solid state, or an optoisolator preferably comprising a photodiode/photocell pair. In this way, the potential of the generator 4 remains uncoupled from the potential of the microprocessor 13, making the latter unaffected by any fluctuations. The isolation obtained through an optoisolator is advantageous from the point of view of energy consumption.

The microprocessor 13 is furthermore connected to a current sensor 15 and to the first transmission/reception means 7.

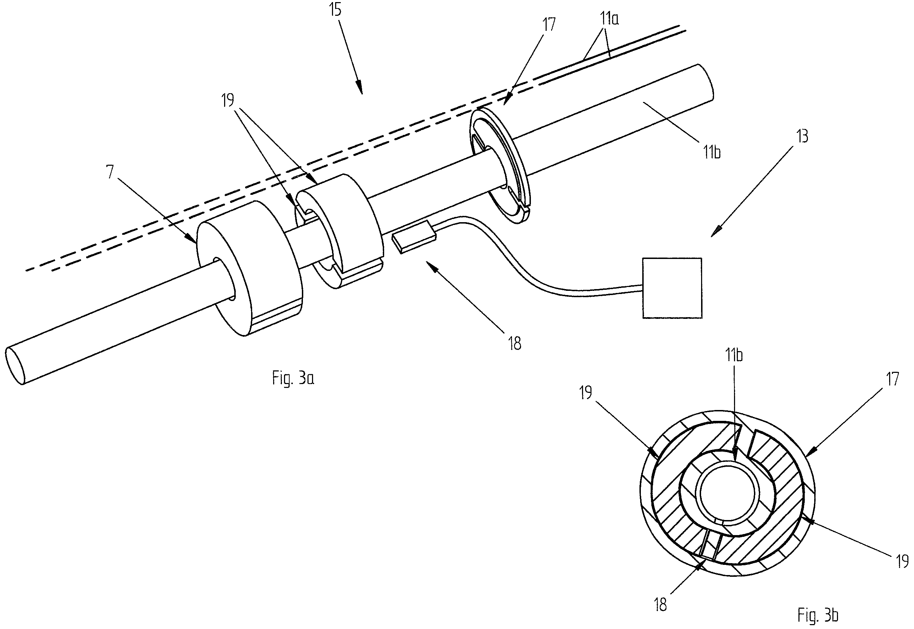

The current sensor 15 makes it possible to detect the presence of welding current supplied by the generator 4 in the cable bundle 11 and to measure its intensity. Its components are better described with reference to Figures 3A and 3B.

The microprocessor 13 thus receives information from the current sensor 15 when current starts flowing through the power supply cables 11b of the cable bundle 11. If the moment when the welding current starts flowing is known, the microprocessor 13 is capable of calculating the total interval of time during which the welding current flows through the cable bundle 11.

The first transmission/reception means 7 make it possible to communicate the data received, processed and/or transmitted by the microprocessor 13 to the other components of the welding system, in particular to the protection unit.

The first transmission/reception means 7 preferably comprise a radiofrequency unit. Said radiofrequency unit is preferably of the Bluetooth® type. The first transmission/reception means 7 thus preferably operate in wireless mode and therefore make the structure of the entire welding system lighter.

If the first transmission/reception means 7 are on and/or are connected to other transmission/reception means, this is properly signalled by the warning lights 34 on the control panel 33 of the control means 6.

Figure 3 schematically shows a 3D view (Figure 3A) and a sectional view (Figure 3B) of a detail of the welding system shown in Figure 1.

More particularly, Figure 3 shows the components of the current sensor 15 of the control system 6.

Figure 3A shows a 3D view of the current sensor 15, which is suited to detect the presence of current flowing through the cable bundle 11 and in particular the one or more power supply cables 11b and to measure its intensity.

The current sensor 15 preferably comprises a ferromagnetic circuit 19 cooperating with a Hall sensor 18.

The ferromagnetic circuit 19 comprises a C-shaped element arranged in such a way as to at least partially envelope one or more power supply cables 11b of the cable bundle 11. As an alternative, the ferromagnetic circuit 19 may comprise two C-shaped elements arranged in such a way as to completely envelop one or more power supply cables 11b of the cable bundle 11.

The Hall sensor 18 is positioned between two adjacent C-shaped elements.

The ferromagnetic circuit 19 is protected by a closing container 17.

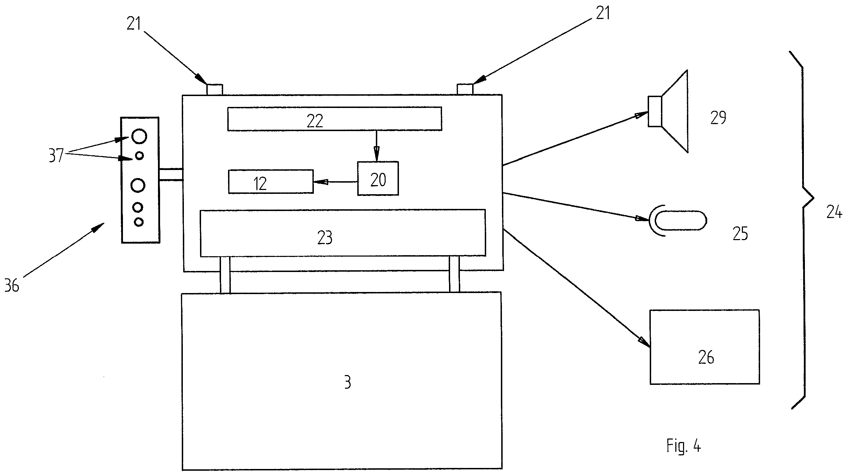

The current sensor 15 furthermore communicates to the microprocessor 13 the presence of current and its intensity, thus allowing the microprocessor 13 to automatically recognize the operating mode of the generator 4: 2T or 4T mode. This will be useful to determine the darkening mode of the protection screen 3 included in the protection unit, as explained here below with reference to Figure 4. In this way, the operator is not required to perform additional settings of the system, since the operating mode is recognized automatically through the reading of the welding current and based on the status of the torch button (8).

The use of a Hall effect current sensor 18 associated with a ferromagnetic circuit 19 constituted by two C-shaped cores positioned around the power supply cables 11b belonging to the cable bundle 11 makes it possible to achieve an optimal compromise between sensitivity to low currents and system compactness. In this way, the sensor occupies a minimal space and can be contained inside the control system 6. Furthermore, this solution offers greater sturdiness and higher immunity to the disturbances that may be coupled and high susceptibility to voltage spikes generated by quick-changing differences in potential.

Figure 3B shows the sensor 15 along a cross section that is perpendicular to the cable bundle 11.

Figure 4 schematically shows a detail of the welding system shown in Figure 1. More particularly, Figure 4 shows a block diagram of the components included in the protection unit. The protection unit, as better described below, allows the operator to protect his/her eyes from the radiation produced by the welding arc 10.

The protection unit preferably comprises a protection helmet 2 (not shown in the figure but visible in Figure 1) that can be worn by the operator during the welding process.

When the operator pushes the button 8 of the torch 1 to start the welding operations, the signal indicating that current is flowing through the one or more control cables 11a belonging to the cable bundle 11 reaches the first microprocessor 13. Using the first transmission/reception means 7 included in the control system 6 it is possible to transmit the signal indicating that the button 8 has been pressed - and that, therefore, the operator intends to start the welding operations - to the second transmission/reception means 12 included in the protection unit.

The second transmission/reception means 12 preferably comprise a radiofrequency unit. Preferably, said radiofrequency unit is of the Bluetooth® type. Therefore, the second transmission/reception means 12 preferably operate in a wireless mode and thus make the structure of the entire welding system lighter. Therefore, through the control system 23 of the protection screen 3, the second microprocessor 20 communicates to the protection screen 3 that the darkening phase can be started.

Once the screen 3 has been darkened, the informaton is transferred from the protection unit to the control system 6. At this point the microprocessor 13 informs the generator 4 that the button 8 has been pressed. Only at this point the generator 4 will be informed of the position of the button 8 and will start supplying current for the welding process.

Thus, the screen 3 will be dark before the supply of welding current by the generator 4 and therefore before the formation of the welding arc 10. In this way, the operator's eyes are never exposed to the radiation emitted by the welding arc 10.

The protection screen 3 preferably comprises an LCD filter. As is known to the expert in the art, an LCD filter is substantially constituted by several layers comprising polaroid films, glass and LCDs. An LCD filter filters and dims light at certain wavelenghts and, changing the control voltage Vc applied to it, it is possible to vary the degree of light filtering/dimming for the different wavelengths, in particular for the wavelengths in the visible spectrum. The degree of darkening (the term "darkening" means the filtration and/or attenuation of the radiation at a certain wavelength) of an LCD filter is measured in DIN degrees.

The desired DIN degree is supplied by the operator as an input, for example through the buttons 37 on the control panel 36 of the protection unit. Alternatively, the DIN degree can be set automatically by the microprocessor 20 that measures the light radiation emitted by the welding arc by means of the sensor 21 and converts it in the required DIN degree based on a table stored in the microprocessor 20 and/or in a memory unit, thus obtaining the control voltage Vc of the LCD filter.

The value of the control voltage Vc that the control system 23 calculates as an input for the protection screen 3 depends on the welding parameters and on some environmental parameters like, for example, the temperature of the protection unit. The temperature sensor 23, in fact, measures the temperature of the protection unit, in particular the temperature of the protection screen 3. In this way, the setting of the control voltage Vc to be applied to the protection screen 3 to determine its correct darkening degree in DIN takes in consideration the actual temperature of the protection screen 3.

More particularly, for each DIN degree selected by the operator and communicated to the protection unit, the second microprocessor 20 has at its disposal a table in which each DIN degree corresponds to a set of possible values of the applicable control voltage Vc. Even more particularly, for each temperature interval within which the temperature of the LCD filter is included, there is a corresponding specific control voltage Vc, for each DIN degree. Therefore, the second microprocessor 20, once having received the value of the temperature of the LCD filter from the temperature sensor 23, verifies the temperature range within which the filter temperature is included and then applies the corresponding control voltage Vc, based on the DIN degree selected by the operator.

The higher the temperature of the LCD filter, the lower the value of the control voltage Vc to be applied and, therefore, the higher the energy saving. Vice versa, the lower the temperature of the LCD filter, the higher the value of the control voltage Vc to be applied and, therefore, the higher the energy consumption. When the operator wants to interrupt the welding operations, the information is transmitted to the protection unit through the transmission/reception means 7. The opening (that is, the reduction in the degree of filtration/attenuation of the radiation) of the protection screen 3, however, does not take place immediately, at the exact moment when the button is released, but with a certain delay with respect to this event. The delay with which the opening of the protection screen 3 takes place is calculated based on the welding time, that is, based on the time during which the current sensor 15 has measured the passage of current inside the cable bundle 11. In any case, the delay never exceeds a preset time, for example 1 sec.

In the case where communication with Bluetooth® technology between the first transmission/reception means 7 and the second transmission/reception means 12 should not work properly due to any technical problem, the darkening of the protection screen 3 is ensured, in any case, by a system of optical sensors 21 included in the protection unit. The optical sensors 21, in fact, indepedently of the communication system described above, detect the presence of the welding radiation and, through the reading system 22 of the optical sensor, communicate to the protection screen 3 that the darkening phase can be started.

The protection unit may comprise, furthermore, transducer means 24. The transducer means 24 comprise, for example, a display unit 26, a microphone 25 and/or an acoustic diffuser 29 intended to allow the operator to communicate with the main unit and/or the external environment, for example the company's data network.

Using the display unit 26, the operator can read the data sent by the main unit and thus get to know the data relating to the welding and/or cutting process. Using the microphone 25 and/or the acoustic diffuser 29, the operator can also communicate with the external environment in relation to both the welding and/or cutting data and any necessary service communication.

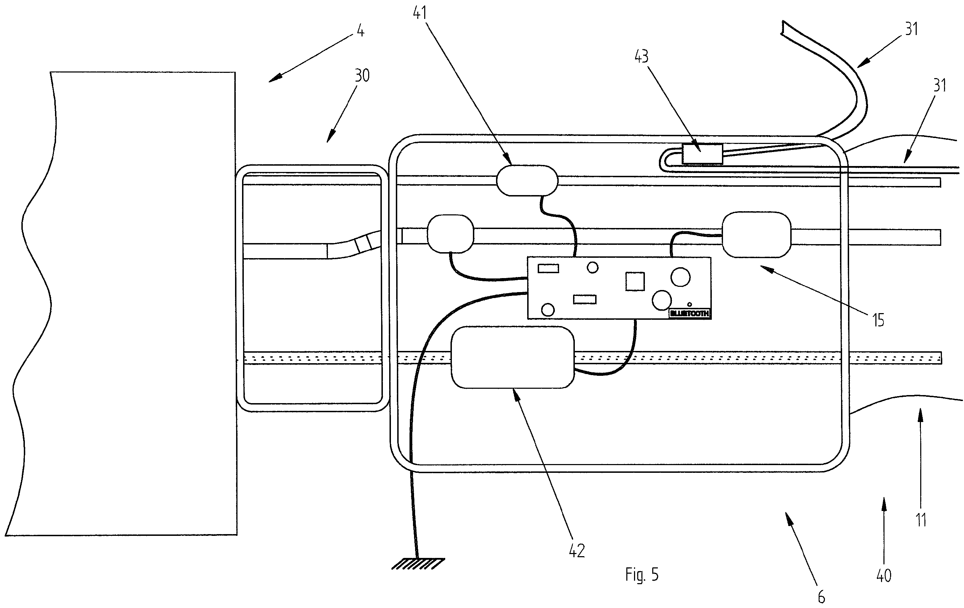

Figure 5 schematically shows a further detail of a welding and/or cutting system. More particularly, Figure 5 schematically shows the data collection/transmission unit.

The data collection/transmission unit mainly comprises a data collection system 27 that in the preferred embodiment shown in the figures is included in the control system 6, as well as a set of sensors 41, 42, 43, 15.

The sensors 41, 42, 43, 15 are preferably included in the connection element 40 that comprises also the power supply cable 11b, the wire guide sheath with the welding wire, the water pipe of the cooling system and the gas pipe coming from the cylinder 28.

The sensors 41, 42, 43, 15, conveniently positioned in the welding system, supply the data collection system 27 with data concerning the wire speed, the flow rate of the gas emitted by the gas cylinder 28, the welding voltage and the flow rate and temperature of the liquid circulating in the torch cooling circuit, where provided.

The sensors communicate with the data collection/transmission system 27 via a wired or wireless connection.

The sensor 41 that measures the gas flow rate can be a differential pressure sensor and/or an absolute pressure sensor, in the case where the geometry of the gas pipe is known. Alternatively, a volumetric flow sensor can be used.

The sensor 43 that measures the liquid flow rate in the cooling circuit can be a differential pressure sensor and/or an absolute pressure sensor, in the case where the geometry of the water pipe is known. Alternatively, a volumetric flow sensor can be used.

The liquid temperature is measured by a temperature sensor of the ptc and ntc type that transduces the temperature into an electrical signal.

The data collection/transmission system 27 collects and/or reads and/or stores the welding data that are transmitted to it. The data collection/transmission system 27 may also comprise software for processing the received data.

The data collected by the data collection/transmission system 27 can be transmitted, via a wired or wireless connection, to the microprocessor 13 included in the control system 6, to the protection unit and/or to the external environment, for example the company's data network. In this way, the operator can be instantaneously informed on the welding parameters that he/she is using, for example by displaying them on the display unit 26 or listening to them through the diffuser 29 located in the helmet 2, and the same data can also be communicated to the external environment. For example, the data can be transmitted to a printing unit in order to print the welding report.

According to the embodiment illustrated herein, the data collection/transmission system 27 is positioned inside the control system 6.

If communication with the data collection/transmission system 27 takes place through Bluetooth® technology, the welding data can be easily received by any device suitable for this type of wireless communication, for example a PC, a tablet or similar devices.

Consequently, the invention is not limited to the embodiments described above, but it is limited only by the scope of protection defined by the attached claims.

## Claims

1. Welding and/or cutting unit comprising:
- a generator (4),
- a welding and/or cutting torch (1),
- a connection element (40) connecting the generator (4) to the welding and/or cutting torch (1), the connection element (40) comprising a connector (30) and one or more power supply cables (11b) of said torch (1), and
- a control system (6) comprising a sensor (15) suited to detect the passage of current,
**characterized in that**
the control system (6) is mounted between the connector (30) and the power supply cable (11a), and the sensor (15) is suited to detect the passage of current in at least one of said one or more cables of said connection element (40); and
said sensor (15) comprises a ferromagnetic circuit (19) that at least partially envelops said at least one of said one or more cables of said connection element (40) and a Hall sensor (18).

2. Welding and/or cutting unit according to claim 1 **wherein** said sensor (15) is suited to measure the intensity of said current that flows through said one or more power supply cables (11b).

3. Welding and/or cutting unit according to any of the preceding claims, **wherein** said ferromagnetic circuit (19) comprises one or more C-shaped elements.

4. Welding and/or cutting unit according to claim 3, **wherein** said one or more C-shaped elements at least partially envelop/envelops said one or more power supply cables (11b) of said torch (1) or parts of said torch body (1).

5. Welding and/or cutting unit according to any of the preceding claims, **characterized in that** it comprises a closing container (17) in which said ferromagnetic circuit (19) is closed.

6. Welding and/or cutting unit according to any of the preceding claims, wherein the control system (6) further comprises a microprocessor (13), and **wherein** said sensor is suited to be connected to the microporcessor (13), in such a way as to signal to said first microprocessor (13) the passage of current in said one or more power supply cables (11b) of said torch (1).

7. Welding and/or cutting unit according to claim 6, wherein the welding and/or cutting torch (1) comprises a button (8), and the microprocessor (13) is suited to automatically recognize the operating mode of said torch (1), for example 2T or 4T mode, based on the signal received from said current sensor (15) and on the position of the button (8).

8. Welding and/or cutting system comprising a protection unit and a welding unit according to any of the preceding claims.

9. Welding and/or cutting system according to claim 8, **characterized in that** in use, the darkening of a protection screen (3), for example an LCD filter, included in said protection unit, is a function of the current measured by said current sensor (15).

## Patentansprüche

1. Schweiß- und/oder Schneideeinheit, Folgendes umfassend:
- einen Generator (4),
- einen Schweiß- und/oder Schneidbrenner (1),
- ein Verbindungselement (40), das den Generator (4) mit dem Schweiß- und/oder Schneidbrenner (1) verbindet, wobei das Verbindungselement (40) einen Verbinder (30) und ein oder mehrere Stromversorgungskabel (11b) des besagten Brenners (1) umfasst, und
- ein Steuerungssystem (6), einen Sensor (15) umfassend, dazu geeignet, den Durchfluss von Strom zu erfassen,
**dadurch gekennzeichnet, dass**
das Steuerungssystem (6) zwischen dem Verbinder (30) und dem Stromversorgungskabel (11a) montiert ist, und der Sensor (15) dazu geeignet ist, in wenigstens einem des/der besagten einen oder mehreren Kabels/Kabel des besagten Verbindungselements (40) den Durchfluss von Strom zu erfassen; und der besagte Sensor (15) einen ferromagnetischen Kreis (19), der wenigstens teilweise das besagte wenigstens eine des/der besagten einen oder mehreren Kabels/Kabel des besagten Verbindungselements (40) umgibt, und einen Hall-Sensor (18) umfasst.

2. Schweiß- und/oder Schneideeinheit nach Patentanspruch 1, **wobei** der besagte Sensor (15) dazu geeignet ist, die Stärke des besagten, durch das besagte eine oder die besagten mehreren Stromversorgungskabel (11b) fließenden Stroms zu messen.

3. Schweiß- und/oder Schneideeinheit nach einem jeden der vorstehenden Patentansprüche, **wobei** der besagte ferromagnetische Kreis (19) ein oder mehrere C-förmige Element/Elemente umfasst.

4. Schweiß- und/oder Schneideeinheit nach Patentanspruch 3, **wobei** das besagte eine oder die besagten mehreren, C-förmigen Element/Elemente wenigstens teilweise das besagte eine oder die besagten mehreren Stromversorgungskabel (11b) des besagten Brenners (1) oder Teile des besagten Brennerkörpers (1) umgibt/umgeben.

5. Schweiß- und/oder Schneideeinheit nach einem jeden der vorstehenden Patentansprüche, **dadurch gekennzeichnet, dass** sie einen Verschlussbehälter (17) umfasst, in welcher der besagte ferromagnetische Kreis (19) umschlossen ist.

6. Schweiß- und/oder Schneideeinheit nach einem jeden der vorstehenden Patentansprüche, wobei das Steuerungssystem (6) des Weiteren einen Mikroprozessor (13) umfasst, und **wobei** der besagte Sensor dazu geeignet ist, derart an den Mikroprozessor (13) angeschlossen zu werden, dass er dem besagten ersten Mikroprozessor (13) den Durchfluss von Strom in dem/den besagten einen oder mehreren Stromversorgungskabel(n) (11b) des besagten Brenners (1) signalisiert.

7. Schweiß- und/oder Schneideeinheit nach Patentanspruch 6, wobei der Schweiß- und/oder Schneidbrenner (1) einen Knopf (8) umfasst, und der Mikroprozessor (13) dazu geeignet ist, automatisch den Betriebsmodus des besagten Brenners (1) zu erkennen, beispielsweise 2T- oder 4T-Modus, basierend auf dem von dem besagten Stromsensor (15) empfangenen Signal und auf der Position des Knopfes (8).

8. Schweiß- und/oder Schneidesystem, eine Schutzeinheit und eine Schweißeinheit nach einem jeden der vorstehenden Patentansprüche umfassend.

9. Schweiß- und/oder Schneidesystem nach Patentanspruch 8, **dadurch gekennzeichnet, dass** während des Betriebs die Verdunkelung eines Schutzschirms (3), beispielsweise eines LCD Filters, der in der besagten Schutzeinheit enthalten ist, eine Funktion des durch den besagten Stromsensor (15) gemessenen Stroms ist.

## Revendications

1. Unité de soudure et/ou de coupe comprenant :
- un générateur (4),
- un chalumeau de soudure et/ou de coupe (1),
- un élément de raccordement (40) reliant le générateur (4) chalumeau de soudure et/ou de coupe (1), l'élément de raccordement (40) comprenant un connecteur (30) et un ou plusieurs câbles d'alimentation (11b) dudit chalumeau (1), et
- un système de contrôle (6) comprenant un capteur (15) indiqué pour détecter le passage de courant,
**caractérisé en ce que**
le système de contrôle (6) est monté entre le connecteur (30) et le câble d'alimentation (11a) et le capteur (15) est indiqué pour détecter le passage de courant dans au moins un desdits un ou plusieurs câbles dudit élément de raccordement (40) ; et
ledit capteur (15) comprend un circuit ferromagnétique (19) qui enroule au moins partiellement ledit au moins un desdits un ou plusieurs câbles dudit élément de raccordement (40) et un capteur de Hall (18).

2. Unité de soudure et/ou de coupe selon la revendication 1, **où** ledit capteur (15) est indiqué pour mesurer l'intensité dudit courant qui s'écoule à travers lesdits un ou plusieurs câbles d'alimentation (11b).

3. Unité de soudure et/ou de coupe selon l'une quelconque des revendications précédentes, **où** ledit circuit ferromagnétique (19) comprend un ou plusieurs éléments en forme de C.

4. Unité de soudure et/ou de coupe selon la revendication 3, **où** lesdits un ou plusieurs éléments en forme de C enroule/enroulent, au moins partiellement, lesdits un ou plusieurs câbles d'alimentation (11b) dudit chalumeau (1) ou des parties dudit corps de chalumeau (1).

5. Unité de soudure et/ou de coupe selon l'une quelconque des revendications précédentes, **caractérisée en ce qu'**elle comprend un boîtier de fermeture (17) dudit circuit ferromagnétique (19).

6. Unité de soudure et/ou coupe selon l'une quelconque des revendications précédentes, où ledit système de contrôle (6) comprend également un microprocesseur (13), et **où** ledit capteur est indiqué pour être relié au microprocesseur (13), de manière à signaler audit premier microprocesseur (13) le passage de courant dans lesdits un ou plusieurs câbles d'alimentation (11b) dudit chalumeau (1).

7. Unité de soudure et/ou de coupe selon la revendication 6, où le chalumeau de soudure et/ de coupe (1) comprend un bouton (8), et le microprocesseur (13) est indiqué pour reconnaître automatiquement le mode de fonctionnement dudit chalumeau (1), par exemple mode 2T o 4T, en fonction du signal reçu dudit capteur de courant (15) et à la position du bouton (8).

8. Système de soudure et/ou de coupe comprenant une unité de protection et une unité de soudure selon l'une quelconque des revendications précédentes.

9. Système de soudure et/ou de coupe selon la revendication 8, **caractérisé en ce qu'**en usage, l'obscurcissement d'un écran de protection (3), par exemple un filtre LCD, compris dans ladite unité de protection, est une fonction du courant mesuré par ledit capteur de courant (15).
